# EUROPEAN PATENT SPECIFICATION

(11) **EP 1 715 835 B1**
(45) Date of publication and mention of the grant of the patent: **27.02.2013**
(21) Application number: 04798365.5
(22) Date of filing: 03.11.2004
(51) Int. Cl.: A61K 8/22, A61K 8/73, A61Q 11/00

(54) **Tooth-whitening composition**
Zahnbleichzusammensetzung
Composition de blanchiment de dents

(30) Priority: 03.11.2003 GB 0325579; 03.08.2004 GB 0417280
(43) Date of publication of application: 02.11.2006
(73) Proprietor: Medtrade Products Ltd., Crewe, Cheshire CW1 6GL (GB)
(72) Inventor: Hardy, Craig, Audlem, Cheshire CW3 0AY (GB)
(74) Representative: Wilson Gunn
(86) International application number: PCT/GB2004/004635
(87) International publication number: WO 2005/044209

(56) References cited:
- EP-A- 0 511 782
- WO-A-01/68045
- WO-A-03/024415
- WO-A2-2004/041102
- US-A1- 2003 124 065

## Description

The present invention relates to a tooth-whitening composition.

It is widely accepted that teeth become stained as a result of daily life. For example, exposure to any of cigarettes, tea and coffee stains the teeth. Clearly, the more the teeth are exposed to these materials the greater the extent of staining/discoluration of the teeth.

Recently, people are becoming even more interested in their appearance and the whiteness of their teeth has become an important concern for many people. As a result of this a number of tooth pastes now claim to have a teeth-whitening effect. These toothpastes are, on the whole; abrasive and, due to short exposure times, have limited effects.

EP-511 781 describes a liquid polymer composition for tooth bleaching. The liquid composition is applied onto the teeth, whereupon it forms a film.

More recently, chemical formulations have become available which have a tooth-whitening effect. For example, WO 01/68045 discloses multilayer patches which utilise peroxide as an active whitening agent. The active material is associated with a backing or carrier material which is removed from the teeth following treatment.

WO 03/015656 refers to a delivery strip for an oral healthcare substance. The oral healthcare substance can be a whitening agent and the delivery strip is removed from the surface of the teeth following treatment.

These systems increase the contact time of the active material with the stained teeth. However, the carrier material, which protects the gum areas from over exposure to the active material, can be uncomfortable for the user and awkward to remove once treatment is concluded. Therefore, it is desirable to provide an effective tooth-whitening composition that is sufficiently mild so as not to damage the tissue surrounding the teeth, such that it can be applied without the need for a carrier material.

According to the present invention there is provided a tooth-whitening composition comprising at least one whitening agent together with at least one film forming material and a flavoring agent characterised in that the composition is in the form of a preformed film which is water soluble and wherein said film is not associated with a carrier material.

The composition of the present invention is provided in the form of a film which is water soluble and requires no carrier material during use. Furthermore, the composition self-adheres to teeth. Typically, the composition adheres to teeth in 0.5 to 2.0 minutes.

The film may be available in a form whereby the film is shaped such that it corresponds, as far as possible, to the shape of the area to which it is to be applied, for example a strip for application to the incisors.

By water-soluble it is meant a material which dissolves in water to make a solution.

The film is applied to the surface of the teeth and, upon contact with saliva, begins to dissolve. The film is such that dissolution occurs over a period of time ranging from about 30 seconds up to about 120 minutes. Advantageously, this slow dissolution enables the film, and therefore active ingredients contained therein, to contact the teeth for a sufficient period of time to effect tooth-whitening.

Surprisingly, following dissolution a coating is left on the teeth for hours after use, giving a gentle long term effect. The user is unaware of the presence of this coating.

Clearly the lack of a carrier material provides greater ease of use than currently available products as there is no carrier material to remove from the surface of the teeth following treatment. It also makes the product more comfortable to wear and gives the users the ability to talk clearly while the product is in use.

Furthermore, the amount of active ingredient to which the user is exposed is controlled. This is in contrast to prior art systems, where the user may disregard the use directions and leave the carrier material, and therefore the active material, in contact with the teeth for too long. This is not a concern with the compositions of the present invention.

The whitening agent is preferably peroxide based. Any peroxide compound may be used, but especially preferred peroxides include hydrogen peroxide and/or urea peroxide.

The whitening agent preferably constitutes from 0.1% to 50% w/w of the total composition.

Advantageously, the use of peroxide alone as the whitening agent gives rise to a composition that is especially stable and therefore has a long shelf life. This is in contrast to whitening agents such as perchlorates which are present in known tooth-whitening compositions.

It is widely known that tooth whitening agents give rise to tooth sensitivity. Such sensitivity is generally experienced in bouts. Surprisingly, the whitening composition of the present invention has been shown to sensitise teeth substantially less than expected and importantly less than comparable products currently on the market.

Thus, there is provided a tooth whitening composition as described herein which does not sensitise teeth.

The film forming material is such that it is water soluble. The types of film forming material described herein may be used alone or in combination such that the film may display a degree of water solubility.

The film forming materials of the present invention are polymeric materials and their choice is limited only by the requirement that they impart water solubility to the film. Suitable film forming material include any of the following either alone or in combination: polysaccharides, polyvinyl alcohol, polyvinyl pyrrolidone, polyurethane and polyacrylic acid based polymers such as CARBOPOL^{®} and derivatives thereof.

The most preferred film forming materials for use with the present invention are those derived from cellulose. Particularly preferred water-soluble film forming materials are carboxymethyl cellulose and hydroxyethyl cellulose.

The film forming material preferably constitutes from 0.1 to 99.9% w/w of the total composition.

In addition to the whitening agent, typically the film preferably comprises any of the following either alone or in combination: at least one plasticiser, and at least one filler.

Suitable plasticisers for use with the present invention include any of the following either alone or in combination: polyhydric alcohols such as glycerol, propylene glycol, polyethylene glycol, sugar syrups such as fructose, maltose, etc,

The plasticiser(s) preferably constitute from 0 to 80% w/w of the total composition.

Suitable fillers for use with the present invention include by way of example any of the following either alone or in combination: silica, silicones, sodium sulphate, sodium bicarbonate, Pluronic surfactants, fatty acid compounds, sodium laureth sulphate and ethyl cellulose. In addition to acting as a filler the materials can be chosen so they either speed or slow the rate of dissolution.

The filler preferably constitutes from 0 to 80% w/w of the total composition.

The composition described herein comprises a flavouring ingredient and may comprise further additional ingredients such as stabilisers, humectants, thickeners, preservatives and processing aids colourants (for colouring the composition not the teeth), dyes (for colouring the teeth) and mixture thereof. The processing aids include silicone based components such as dimethicone.

It is envisaged that the composition of the present invention will find utility in delivering other benefits to the teeth/mouth. For example, the composition may further comprise any of the following either alone or in combination: antimicrobial ingredients, anti-plaque components, anti-tartar components, teeth cleaning agents, desensitising agents, herbals, honey (delivers sweetening and antimicrobial properties), pharmaceuticals, appetite surpressors and biologically active compounds.

According to a further aspect of the present invention there is provided the use of a tooth-whitening composition as hereinbefore described for reducing discolouration on teeth.

According to a further aspect of the present invention there is provided a method for the manufacture of a tooth-whitening composition as hereinbefore desribed comprising the steps of: preparing an aqueous mixture which comprises at least one whitening agent together with at least one film forming material, casting the said mixture onto a substrate and allowing the mixture to dry.

The whitening agent may be contained within the film forming material prior to its addition to the composition.

In an alternative procedure, a suitable film is pre-prepared and a whitening agent is then applied to its surface. The whitening agent may be applied to the film by way of spraying, pressure and/or heat transfer or dip coating.

Thus, according to a further aspect of the present invention there is provided a method for the preparation of a tooth whitening composition comprising the steps of: applying to the surface of a pre-prepared film comprising a water-soluble film forming material a whitening agent thereby forming a teeth whitening composition.

The substrate on to which the composition is cast may be a coated paper, coated metal or plastic material.

The composition of the present invention may be available in the form of a multi-layered film wherein, for example, the composition comprises a layer of water-dispersible film forming material having a layer of water-soluble film forming material comprising a whitening agent located on at least one side thereof. It is to be understood that there are many ways in which such a layered structure can be constructed. Thus the combinations of components within each layer and the order in which the layers contact each other is limited only by the compatibility of the components within each layer.

In use, a strip of the film of the present invention can be applied to the teeth each morning and each night for 14 days. It might be expected that the application of such a product without a carrier or backing material would irritate or redden the tissues of the mouth; surprisingly it displays no such irritation (see Table 2). Such applications are well below the SCCNFP (Scientific Committee on Cosmetic and Non Food Products) proposed guidelines for exposure to components such as hydrogel peroxide at 50mg / day.

The present invention will now be described further by way of example only.

### Reference Example 1

| **Material** | **Amount (% w/w)** | **Function** |
|---|---|---|
| Sodium Alginate | 60% | Film forming polymer/thickener |
| Hydrogen Peroxide | 7.5% | Whitening agent |
| Fructose Syrup | 2% | Humectant/plasticizer/sweetener |
| Glycerol | 10% | Humectant |
| Sodium Pyrophosphate | 0.25% | Stabiliser |
| Methylparaben | 0.25% | Preservative |
| Ethyl Cellulose | 20% | 2nd film former / Filler |

An additional 3500% of (80% purified water & 20% Ethanol) is used in the manufacture of this example. The methylparben is dissolved in the water while hot and then the mixture is cooled to below 25°C. The ethyl cellulose is dissolve in the ethanol. The water phase and ethanol phases are mixed and added to a low shear mixer and circulated. At this point the other ingredients (excluding the sodium alginate) are added and mixed for a further 30 minutes. The sodium alginate is added slowly to produce a homogenous mixture.

The resulting polymer solution is cast onto a substrate and dried under heat, UV or IR to give a soft flexible water soluble film which can be removed from the substrate and then be cut into pieces for application and whitening of the teeth.

### Example 2

| **Material** | **Amount (% w/w)** | **Function** |
|---|---|---|
| Hydroxyethylcellulose | 75% | Film forming polymer/thickener |
| Hydrogen Peroxide | 5.5% | Whitening agent |
| Propylene Glycol | 5% | Humectant |
| Glycerol | 12% | Humectant |
| Sodium Stannate | 2% | Stabiliser |
| Methylparaben | 0.25% | Preservative |
| Dimethicone | 0.25% | Processing aid |

An additional 3000% of purified water is used in the manufacture of this example. The water added to a low shear mixer and circulated. The methylparben is dissolved in the water and then the mixture is cooled to below 25°C. At this point the other ingredients (excluding the hydroxyethylcellulose) are added and mixed for a further 10 minutes. The hydroxyethylcellulose is added slowly to produce a homogenous mixture.

The resulting polymer solution is cast onto a substrate and dried under heat, UV or IR to give a soft flexible water soluble film which can then be cut into pieces for application and whitening of the teeth. The water soluble film is removed from the substrate prior to application to the teeth.

### Clinical Studies

Clinical studies have been conducted to establish the effectiveness of the composition of the present invention. Table 1, below, shows the Vita^{®} shade scores following treatment with a composition of the present invention.

**Table 1**

| Evaluation Time | Sample Size | Mean baseline Vita score (prior to treatment) | Mean Vita shade score (after treatment) | No. of shade changes from baseline |
|---|---|---|---|---|
| Day 8 | 6 | 4.83 | 3.25 | 1.58 |
| Day 15 | 6 | 4.83 | 2.50 | 2.33 |

The results show that following twice daily application of the tooth whitening composition described herein, after 8 days tooth staining was reduced by around 1.5 shade changes. Following treatment for 15 days tooth staining was further reduced by over 2 shade changes.

The Vita^{®} shade score test is the standard test used to determine the degree of tooth whitening effected by a particular composition. The test and the conditions under which it is conducted are well known to those skilled in the art.

Table 2 give the results of the clinical assessment of teeth sensitivity following use of the composition of the present invention against a commercial control with a carrier. The products were used according to their recommended directions. Sensitivity was reported on a numerical scale by the users under GCP (good clinical practice) conditions. Such testing is well known to those skilled in the art.

**Table 2**

| **Treatment A - (Test Product)** | | | | |
|---|---|---|---|---|
| Evaluation | n | Baseline Score | Mean Score | Mean changes from baseline |
| Day 8 | 12 | 0.00 | 0.00 | 0.00 |
| Day 15 | 12 | 0.00 | 0.00 | 0.00 |

| **Treatment %- (Commercial Control Product)** | | | | |
|---|---|---|---|---|
| Evaluation | n | Baseline Score | Mean Score | Mean changes from baseline |
| Day 8 | 13 | 0.00 | 0.15 | 0.15 |
| Day 15 | 13 | 0.00 | 0.08 | 0.08 |
| Test Product: No of incidences of reported sensitivity = zero | | | | |
| Control Product: No of incidences of reported sensitivity = 3 (23% of test group) | | | | |

It is of course to be understood that the invention is not intended to be restricted to the details of the above embodiments which are described by way of example only.

## Claims

1. A tooth-whitening composition in the form of a preformed film for application to the surface of the teeth, said film being formed from at least one whitening agent together with at least one film forming material, wherein said film is water soluble, and wherein said film is not associated with a carrier material, the tooth-whitening composition further comprising a flavouring ingredient.

2. A composition according to claim 1, wherein the composition further comprises any of the following, either alone or in combination: at least one plasticiser and at least one filler.

3. A composition according to claim 1 or claim 2, wherein the film forming polymer is selected from any of the following, either alone or in combination: polysaccharides, polyvinyl alcohol, polyvinyl pyrrolidone and polyacrylic acid based polymers.

4. A composition according to claim 3, wherein the film forming polymer is derived from cellulose.

5. A composition according to claim 2, wherein the plasticiser is selected from any of the following: polyhydric alcohols and sugar syrups.

6. A composition according to claim 2 or claim 5, wherein the plasticiser constitute from 0 to 80% w/w of the total composition.

7. A composition according to any one of the preceding claims, wherein the whitening agent is peroxide based.

8. A composition according to claim 7, wherein the peroxide is hydrogen peroxide and/or urea peroxide.

9. A composition according to any one of the preceding claim, wherein the whitening agent constitutes from 0.1 % to 50% w/w of the total composition.

10. A composition according to any preceeding claim, wherein the composition further comprises a processing aid being a silicone based compound.

11. The use of a tooth-whitening composition as described in claim 1 for reducing discolouration on teeth.

12. A method for the manufacture of a tooth-whitening composition of claim 1 comprising the steps of: preparing an aqueous mixture which comprises at least one whitening agent together with at least one film forming material and a flavouring ingredient, casting the said mixture onto a substrate and allowing the mixture to dry.

## Patentansprüche

1. Zusammensetzung zum Bleichen von Zähnen in Form einer vorgeformten Folie zum Anwenden auf der Oberfläche der Zähne, wobei die Folie aus wenigstens einem Bleichmittel zusammen mit wenigstens einem folienbildenden Material gebildet ist, wobei die Folie wasserlöslich ist und wobei die Folie nicht mit einem Trägermaterial verbunden ist, und wobei die Zusammensetzung zum Bleichen von Zähnen ferner einen Geschmacksstoff umfasst.

2. Zusammensetzung nach Anspruch 1, wobei die Zusammensetzung ferner eines der folgenden Mittel entweder allein oder in Kombination umfasst: wenigstens einen Weichmacher und wenigstens ein Füllmittel.

3. Zusammensetzung nach Anspruch 1 oder Anspruch 2, wobei das folienbildende Polymer aus einem der folgenden Stoffe entweder allein oder in Kombination ausgewählt ist: Polysaccharide, Polyvinylalkohol, Polyvinylpyrrolidon und Polymere auf der Basis von Polyacrylsäure.

4. Zusammensetzung nach Anspruch 3, wobei das folienbildende Polymer aus Zellulose hergeleitet ist.

5. Zusammensetzung nach Anspruch 2, wobei der Weichmacher aus einem der folgenden Stoffe ausgewählt ist: mehrwertige Alkohole und Zuckersirupe.

6. Zusammensetzung nach Anspruch 2 oder Anspruch 5, wobei der Weichmacher 0 bis 80 Gewichtsprozent der gesamten Zusammensetzung ausmacht.

7. Zusammensetzung nach einem der vorhergehenden Ansprüche, wobei das Bleichmittel auf Peroxid basiert.

8. Zusammensetzung nach Anspruch 7, wobei es sich bei dem Peroxid um Wasserstoffperoxid und/oder Harnstoffperoxid handelt.

9. Zusammensetzung nach einem der vorhergehenden Ansprüche, wobei das Bleichmittel 0,1 bis 50 Gewichtsprozent der gesamten Zusammensetzung ausmacht.

10. Zusammensetzung nach einem der vorhergehenden Ansprüche, wobei die Zusammensetzung ferner ein Verarbeitungshilfsmittel umfasst, bei dem es sich um eine Verbindung auf der Basis von Silikon handelt.

11. Verwendung einer Zusammensetzung zum Bleichen von Zähnen, wie sie in Anspruch 1 beschrieben ist, zum Reduzieren einer Verfärbung auf Zähnen.

12. Verfahren für die Herstellung einer Zusammensetzung zum Bleichen von Zähnen nach Anspruch 1, welches die folgenden Schritte umfasst: Herstellen eines wässrigen Gemisches, welches wenigstens ein Bleichmittel zusammen mit wenigstens einem folienbildenden Material und einen Geschmacksstoff umfasst, wobei das Gemisch auf ein Substrat gegossen wird und das Gemisch trocknen gelassen wird.

## Revendications

1. Composition de blanchiment des dents sous la forme d'un film préformé pour une application sur la surface des dents, ledit film se composant d'au moins un agent de blanchiment ainsi que d'au moins un matériau formant un film, dans laquelle ledit film est soluble dans l'eau, et dans laquelle ledit film n'est pas associé à un matériau support, la composition de blanchiment des dents comprenant en outre un ingrédient aromatisant.

2. Composition selon la revendication 1, la composition comprenant en outre l'un quelconque des éléments suivants, seul ou en combinaison : au moins un plastifiant et au moins une charge.

3. Composition selon la revendication 1 ou 2, dans laquelle le polymère formant un film est choisi parmi l'un quelconque des éléments suivants, seul ou en combinaison : polysaccharides, alcool polyvinylique, polyvinylpyrrolidone et polymères à base d'acide polyacrylique.

4. Composition selon la revendication 3, dans laquelle le polymère formant un film est dérivé de la cellulose.

5. Composition selon la revendication 2, dans laquelle le plastifiant est choisi parmi l'un quelconque des éléments suivants : alcools polyhydriques et sirops de sucre.

6. Composition selon la revendication 2 ou 5, dans laquelle le plastifiant constitue de 0 à 80 % m/m de la composition totale.

7. Composition selon l'une quelconque des revendications précédentes, dans laquelle l'agent de blanchiment est à base de peroxyde.

8. Composition selon la revendication 7, dans laquelle le peroxyde est le peroxyde d'hydrogène et/ou le peroxyde d'urée.

9. Composition selon l'une quelconque des revendications précédentes, dans laquelle l'agent de blanchiment constitue de 0,1 % à 50 % m/m de la composition totale.

10. Composition selon l'une quelconque des revendications précédentes, la composition comprenant en outre un adjuvant qui est un composé à base de silicone.

11. Utilisation d'une composition de blanchiment des dents selon la revendication 1, afin de réduire la décoloration sur les dents.

12. Procédé de fabrication d'une composition de blanchiment des dents selon la revendication 1, comprenant les étapes consistant à: préparer un mélange aqueux qui comprend au moins un agent de blanchiment ainsi qu'au moins un matériau formant un film et un ingrédient aromatisant, couler ledit mélange sur un substrat et permettre le séchage du mélange.
